# EUROPEAN PATENT SPECIFICATION

(11) **EP 0 932 409 B1**
(45) Date of publication and mention of the grant of the patent: **26.03.2003**
(21) Application number: 97941095.8
(22) Date of filing: 19.09.1997
(51) Int. Cl.: A61K 35/78

(54) **A METHOD FOR PRODUCING AN EXTRACT FROM VALERIANA OFFICINALIS CONTAINING HIGH LEVELS OF VALERENIC ACID**
VERFAHREN ZUR HERSTELLUNG VON EINEM VALERIANA OFFICINALIS EXTRAKT MIT HOHEM GEHALT AN VALERENSÄURE
PROCEDE DE PRODUCTION D'UNE EXTRAIT DE VALERIANE OFFICINALE CONTENANT DES TAUX ELEVES D'ACIDE VALERIQUE

(30) Priority: 28.09.1996 GB 9620291
(43) Date of publication of application: 04.08.1999
(73) Proprietor: Essential Nutrition Ltd., Brough HU15 1EF (GB)
(72) Inventor: WHEATLEY, Gary, William, Hull HU9 4DS (GB); CHAPMAN, Thomas, Brian, Brough HU15 1DY (GB)
(74) Representative: Stuttard, Garry Philip
(86) International application number: GB9702559
(87) International publication number: WO98013054

(56) References cited:
- BE-A- 1 001 776
- M MORVAI-VIT NYI ET AL.: "ANALYSIS OF SUPERCRITICAL FLUID EXTRACTS OF VALERIAN ROOTS BY COMBINED CHROMATOGRAPHY/MASS SPECTROMETRY" ACTA HORTICULTURAE, no. 344, 1993, pages 386-403, XP002052194

## Description

This invention relates to a method of preparation of an extract from the root of Valerian officinalis that contains high levels of the compound valerenic acid. Valerenic acid exhibits sedative activity in. standard animal models.

Valerian root has been traditionally used as a herbal medicine with mild tranquillising and sedative actions. Unlike most traditional herbal medicines there is sufficient evidence of safety and efficacy for Valerian root and preparations derived therefrom to be the subject of an official monograph in the current editions of both the British Pharmacopoeia and the European Pharmacopoeia.

Early scientific opinion held that the essential oil was responsible for the pharmacological actions of Valerian. However, the class of epoxy iridoid esters known as valepotriates were discovered in the 1960s and it was subsequently discovered that these compounds exhibited sedative activity. However, valepotriates are insoluble in water and are not present in aqueous or alcoholic extracts of Valerian root.

The observation that the sedative activity of Valerian root could not be completely accounted for by the presence of the valepotriates stimulated further research and more recently the importance of valerenic acid, a terpenic constituent of the essential oil, has been recognised. Several studies have confirmed the sedative action of this compound in both standard animal models and in enzyme inhibition studies related to key neurochemical pathways. The central role of valerenic acid has been acknowledged by major European manufacturers of pharmacopoeial grade Valerian extract who now standardise their products by reference to the content of valerenic acid or total sesquiterpenes. The official thin layer chromatographic method described in the British Pharmacopoeia for the analysis of Valerian stresses the importance of the presence of both the valepotriates and valerenic acid. The structure of valerenic acid and the valerenic acid and the valepotriates is provided below: 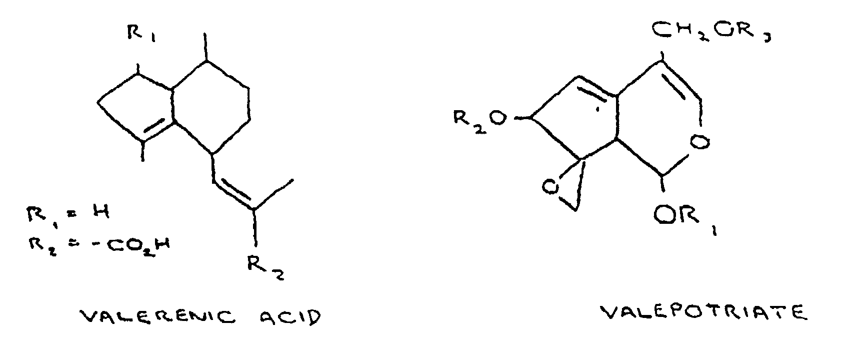
(Valtrate) R₁ = R₂ = COCH₂CH(CH₃)₂ R₃ = COCH₃
(Isovaltrate) R₁ = R₃ = COCH₂CH(CH₃)₂ R₂ = COCH₃
(Acevaltrate I) R₁ = COCH₂C(CH₃)₂OCOCH₃
   R₂ = COCH₂ CH(CH₃)₂
   R₃ = COCH₃
(Acevaltrate II) R₁ = COCH₂CH(CH₃)₂
   R₂ = COCH₂C(CH₃)₂OCOCH₃
   R₃ = COCH₃

In recent years extraction using liquified carbon dioxide has been applied to production of fractions rich in biologically active compounds, particularly terpenoids, from plant based raw materials.

Liquid carbon dioxide has a high selectivity, being able to solubilise low molar mass compounds of moderate polarity whilst leaving behind in the matrix higher molecular weight lipids, waxes and pigments which would otherwise increase the bulk of an extract and dilute the actives content. Liquid carbon dioxide is also superior to non-polar organic solvents in that it is non-flammable, so that the solvent can be safely vented to the atmosphere avoiding waste disposal and recycling costs. The intrinsically non-toxic and highly volatile nature of carbon dioxide avoids any problems of elimination of residual levels of harmful solvents from the product.

According to the present invention there is provided a method of preparation of an extract containing an amount of more than 4wt% of valerenic acid comprising the step of extraction of the root of *Valerian officinalis* with subcritical carbon dioxide and allowing the carbon dioxide to evaporate from the resultant mixture.

The resultant residual extract contains a higher percentage of valerenic acid than may be obtained by solvent extraction using aqueous alcoholic mixtures. The extract also exhibits *in vivo* sedative activity.

A cosolvent may be employed for example a polar hydroxylic solvent such as C₁ to C₄ alcohol, preferably ethanol. An amount of cosolvent of 5 to 20%, preferably 10% by weight may be employed.

Preferred methods involve extraction at a pressure 96 to 276 bars (1400 to 4000 psi), preferably 100 bars (1500 psi) may be employed.

Preferred methods in accordance with this invention do not employ a cosolvent as this has been found to give proportions of valerenic acid greater than 10%.

The invention is further described by means of example but not in any limitative sense.

### Example

Roughly ground Valerian root was packed into a suitable pressured vessel. A volume of liquid carbon dioxide at the ratio of approximately 10ml of liquid carbon dioxide per 1g of herb was allowed to pass through the raw material at a flow rate of approximately 2ml/min. The liquid carbon dioxide was then collected, the pressure released and carbon dioxide allowed to vent to the atmosphere. Removal of any added cosolvent or residual moisture was completed by drying in a vacuum desiccator. The residual extract in the collection vessel was an oil or semi-solid depending on the exact extraction conditions.

The following range of extraction conditions were employed:

| SAMPLE | PRESSURE | TEMP | COSOLVENT | %YIELD | % VALERENIC ACID |
|---|---|---|---|---|---|
| Val 1 | 100 bars (1500 psi) | 25°C | None | 1.1% | 10.5%w/w |
| Val 2 | 96 bars (1440 psi) | 36°C | None | 1.0% | 10.8% |
| Val 3 | 100 bars (1500 psi) | 25°C | 10% Ethanol | 4.5% | 5.3% |
| Val 4 | 276 bars (4000 psi) | 36°C | None | 1.0% | 10.8% |

The valerenic acid content was determined by an HPLC method based on that of R Hansel & J Schulz, Deutsche Apotheker Zeitung, 122, 215 - 219 (1982).

Sub-critical liquid CO₂ (25°C / 100 bars (1500 psi)) liquid CO₂ close to the critical temp (36°C / 96 bars (1440 psi)) and supercritical CO₂ significantly above the critical temperature (36°C / 276 bars (4000 psi)) all gave products containing useful proportions of valerenic acid. The extraction method for Val 2 and Val 4 are provided for the purposes of comparison only with an embodiment of the extraction method of the invention carried out on Val 1 and Val 3.

The addition of 10% ethanol cosolvent has been found to increase the overall yield of extract but significantly decreased the content of valerenic acid as other more polar compounds were also extracted.

## Claims

1. A method of preparation of an extract containing an amount of more than 4 wt% of valerenic acid comprising the step of extraction of the root of *Valerian officinalis* with subcritical carbon dioxide and allowing the carbon dioxide to evaporate from the resultant mixture.

2. A method as claimed in claim 1, wherein the root of *Valerian officinalis* is extracted with a mixture of subcritical liquid carbon dioxide and a polar hydroxylic cosolvent.

3. A method as claimed in claim 2, wherein the polar hydroxylic cosolvent is a C₁ - C₄ alcohol.

4. A method as claimed in claim 3, wherein the alcohol is ethanol.

5. A method as claimed in any of claims 2 to 4, wherein the amount of cosolvent is 5 to 20% by weight.

6. A method as claimed in claim 5, wherein the amount of cosolvent is 10% by weight.

7. A method as claimed in any preceding claim, wherein the extraction is carried out at a pressure of 96 to 276 bars (1400 to 4000 psi).

8. A method as claimed in claim 7, wherein the extraction is carried out at a pressure of 100 bars (1500 psi).

9. A method as claimed in claim 1 wherein the extract contains an amount of more than 10% by weight of valerenic acid.

## Patentansprüche

1. Verfahren zur Herstellung eines Extrakts, welcher eine Menge von mehr als vier Gewichtsprozent Valerensäure enthält, umfassend die Stufe der Extraktion der Wurzel von Valerian officinalis mit subkritischem Kohlendioxid und des Verdampfens des Kohlendioxids aus dem resultierenden Gemisch.

2. Verfahren nach Anspruch 1, worin die Wurzel von Valerian officinalis mit einem Gemisch aus subkritischem flüssigen Kohlendioxid und einem polaren hydroxylischen Co-Lösungsmittel extrahiert wird.

3. Verfahren nach Anspruch 2, worin das polare hydroxylische Co-Lösungsmittel ein C₁-C₄-Alkohol ist.

4. Verfahren nach Anspruch 3, worin der Alkohol Ethanol ist.

5. Verfahren nach einem der Ansprüche 2 bis 4, worin die Menge an Co-Lösungsmittel 5 bis 20 Gewichtsprozent beträgt.

6. Verfahren nach Anspruch 5, worin die Menge an Co-Lösungsmittel 10 Gewichtsprozent beträgt.

7. Verfahren nach einem der vorhergehenden Ansprüche, worin die Extraktion bei einem Druck von 96 bis 276 bar (1400 bis 4000 psi) durchgeführt wird.

8. Verfahren nach Anspruch 7, worin die Extraktion bei einem Druck von 100 bar (1500 psi) durchgeführt wird.

9. Verfahren nach Anspruch 1, worin der Extrakt eine Menge von mehr als 10 Gewichtsprozent Valerensäure enthält.

## Revendications

1. Procédé de préparation d'un extrait contenant une quantité de plus de 4% en poids d'acide valérénique comprenant l'étape d'extraction de la racine de *Valerian officinalis* avec du dioxyde de carbone sous-critique, et permettant au dioxyde de carbone de s'évaporer du mélange obtenu.

2. Procédé selon la revendication 1, dans lequel la racine de *Valerian officinalis* est extraite avec un mélange de dioxyde de carbone liquide sous-critique et d'un co-solvant hydroxylique polaire.

3. Procédé selon la revendication 2, dans lequel le co-solvant hydroxylique polaire est un alcool en C₁ à C₄.

4. Procédé selon dans la revendication 3, dans lequel l'alcool est l'éthanol.

5. Procédé selon l'une quelconque des revendications 2 à 4, dans lequel la quantité de co-solvant est de 5 à 20% en poids.

6. Procédé selon la revendication 5, dans lequel la quantité de co-sotvant est de 10% en poids.

7. Procédé selon l'une quelconque des revendications précédentes, dans lequel l'extraction est effectuée à une pression de 96 à 276 bars (1400 à 4000 psi).

8. Procédé selon la revendication 7, dans lequel l'extraction et effectuée à une pression de 100 bars (1500 psi).

9. Procédé selon la revendication 1, dans lequel l'extrait contient une quantité supérieure à 10% en poids d'acide valérénique.
